# EUROPEAN PATENT SPECIFICATION

(11) **EP 3 074 079 B1**
(45) Date of publication and mention of the grant of the patent: **15.01.2020**
(21) Application number: 14808795.0
(22) Date of filing: 20.11.2014
(51) Int. Cl.: A61M 25/09

(54) **MEDICAL DEVICES FOR ACCESSING BODY LUMENS**
MEDIZINISCHE VORRICHTUNGEN FÜR DEN ZUGANG ZU KÖRPERLUMEN
DISPOSITIFS MEDICAUX PERMETTANT D'ACCEDER A DES LUMIERES CORPORELLES

(30) Priority: 26.11.2013 US 201361908843 P
(43) Date of publication of application: 05.10.2016
(73) Proprietor: Boston Scientific Scimed, Inc., Maple Grove, MN 55311 (US)
(72) Inventor: ABNER, William, Marlborough, Massachusetts 01752 (US)
(74) Representative: Vossius & Partner Patentanwälte Rechtsanwälte mbB
(86) International application number: PCT/US2014/066665
(87) International publication number: WO 2015/080948

(56) References cited:
- US-A- 5 916 178
- US-A1- 2006 089 568
- US-A1- 2013 110 000
- US-B1- 7 717 864

## Description

### Technical Field

The present disclosure pertains to medical devices, and methods for manufacturing medical devices. More particularly, the present disclosure pertains to medical device for accessing body lumens.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

US 2006/0089568 A1 discloses a guidewire for medical use such as in vascular and nonvascular systems. The distal end of the guidewire is of a smaller diameter and softer than the proximal end and fitted with a coil for springiness such that the distal end will bend when encountering curves in the body passageways. The distal end may also be tapered to provide an additional gradient of softness.

US 7 717 864 B1 discloses a guidewire that is formed, at least in part, of a composite elongate core formed, at least in part, of precipitation hardened material.

US 5 916 178 A discloses a guidewire for use in a catheter comprising a unitary core wire, a distal tip and an elongate tube. The core wire has a body segment and a transition segment, the body segment being of substantially uniform outer diameter with a distal end serially disposed proximal to the transition segment proximal end, the transition segment being more flexible than the body segment, and progressively reduced in cross-section from the body segment.

US 2013/0110000 A1 discloses a dual diameter guide wire.

### Brief Summary

This disclosure provides design, material, manufacturing method, and use alternatives for medical devices. A medical guide wire according to the invention is defined by the features of claim 1.

Another example medical guidewire may include a core wire comprising a superelastic material and having a longitudinal axis. The core wire may include a distal constant diameter section including a proximal end and a distal end. The distal end may define a distal end of the core wire. The distal constant diameter section may have a length in the range of 0.1cm to 2.5 cm and a diameter in the range of 0.001 inches to 0.008 inches. The core wire may also include a tapered section having a proximal end and a distal end. The distal end may be attached to the proximal end of the distal constant diameter section such that a first inflection point is defined where the distal end of the tapered section and the proximal end of the distal constant diameter section meet. The tapered section may have a length in the range of 0.5cm to 3cm. The core wire may also include a proximal portion having a distal end attached to the proximal end of the tapered section. The core wire may be configured such that when a predetermined longitudinal force is applied to the distal end of the distal constant diameter section along the longitudinal axis, the distal constant diameter section prolapses such that a loop is defined about the inflection point.

Methods for gaining access to an opening in a body lumen are also disclosed, however these methods are not a part of the present invention. The methods may include providing or otherwise using a guidewire such as the guidewires disclosed herein. The method may also include contacting a distal end of the guidewire with tissue in or adjacent to the opening, applying a first predetermined longitudinal force to the distal end of the core wire along the longitudinal axis such that a loop is formed in the guidewire, and advancing the loop formed in the guidewire into the opening.

Example medical devices for accessing an opening to a body lumen are also disclosed. An example medical device may include an elongate shaft having a distal constant diameter section, a first tapered section attached to the distal constant diameter section, an intermediate constant diameter section attached to the first tapered section, a second tapered section attached to the intermediate constant diameter section, and a proximal constant diameter section attached to the second tapered section. The distal constant diameter section may have a length in the range of 0.1cm to 2.5 cm. A first inflection point may be defined in the shaft where the distal constant diameter section and the first tapered section meet. The shaft may be configured to form a first loop at the first inflection point when subjected to a first pre-determined longitudinal force. A second inflection point may be defined in the shaft where the intermediate constant diameter section and the second tapered section meet. The shaft may be configured to form a second loop at the second inflection point when subjected to a second pre-determined longitudinal force. The first predetermined force may be in the range of 20g to 200g. The second predetermined force may be in the range of 250g to 700.

The above summary of some embodiments is not intended to describe each disclosed embodiment or every implementation of the present disclosure. The Figures, and Detailed Description, which follow, more particularly exemplify these embodiments. References to embodiments throughout the description which are not under the scope of the appended claims merely represent possible exemplary executions and are therefore not part of the present invention.

### Brief Description of the Drawings

The disclosure may be more completely understood in consideration of the following detailed description in connection with the accompanying drawings, in which:
Figure 1 is a partial cross-sectional side view of an example guidewire;
Figure 2 is a side view of an example core wire;
Figure 3 is a side view of an example core wire with a loop formed therein at a first deflection point;
Figure 4 is a side view of an example core wire with a loop formed therein at a second deflection point;
Figures 5-7 are plan views depicting an example guidewire being advanced to a target region.

### Detailed Description

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

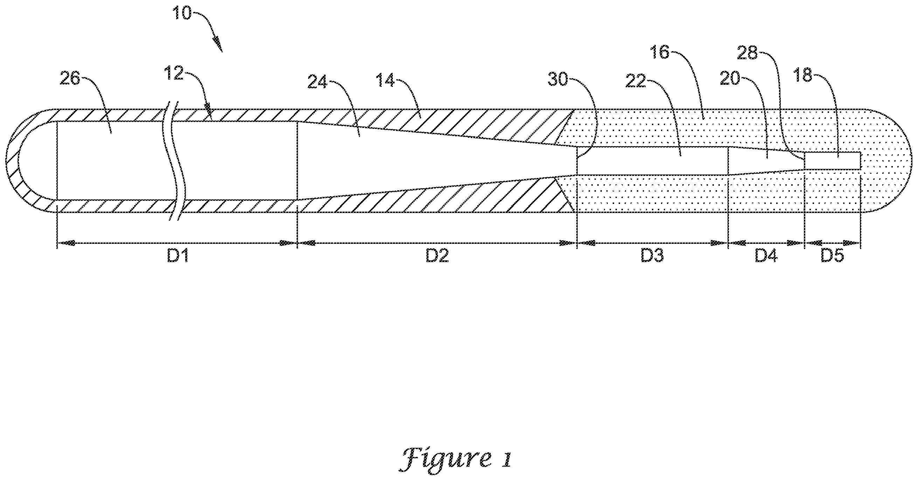

Figure 1 is a partial cross-sectional side view of an example guidewire 10. Guidewire 10 may include a core wire 12. A sheath 14 may be disposed along at least a portion of core wire 12. In some embodiments, a tip member 16 may be disposed about a portion of core wire 12.

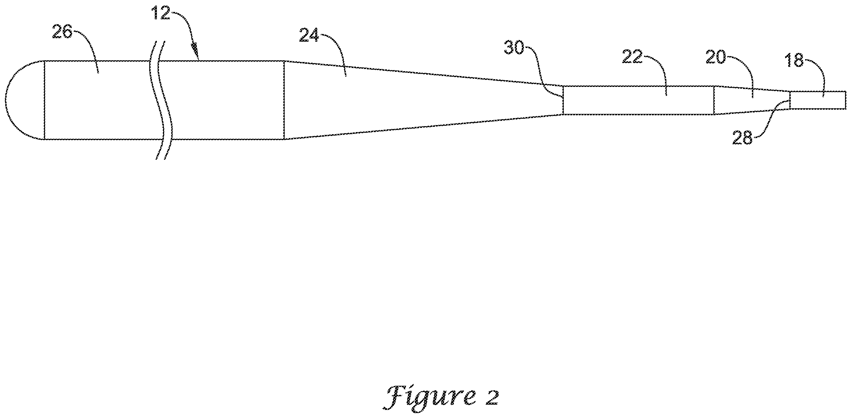

Core wire 12, which can also be seen in Figure 2, may include a first or distal constant diameter section 18. A first tapered section 20 may be coupled to distal constant diameter section 18. For example, a proximal end of distal constant diameter section 18 may be attached to a distal end of first tapered section 20. A second or intermediate constant diameter section 22 may be coupled to first tapered section 20. For example, a proximal end of first tapered section 20 may be attached to a distal end of intermediate constant diameter section 22. A second tapered section 24 may be coupled to intermediate constant diameter section 22. For example, a proximal end of intermediate constant diameter section 22 may be attached to a distal end of second tapered section 24. A third or proximal constant diameter section 26 may be coupled to second tapered section 24. For example, a proximal end of second tapered section 24 may be attached to a distal end of proximal constant diameter section 26. These are just examples. Core wire 12 may include other sections.

In some embodiments, core wire 12 may be a unitary structure or otherwise formed from a single monolith of material. In other embodiments, core wire 12 may be formed from a plurality of different structures that are secured together. This may include one or more of sections 18/20/22/24/26 being separate structures that are secured to the remaining sections of core wire 12. In such embodiments, the one or more separate structures may be secured to remaining sections of core wire 12 using a suitable bonding technique such as welding, brazing, thermal bonding, adhesive bonding, mechanical bonding and/or the use of a mechanical connector, or the like.

The dimensions of core wire 12 may vary. Disclosed herein are some example dimensions for the various sections of core wire 12. These dimensions are meant to be examples and are not intended to be limiting. Other dimensions are contemplated.

In at least some embodiments, proximal constant diameter section 26 may have a length (represented in Figure 1 as dimension D1) of about 200-600cm (78.4-236.2 inches), or about 260-500cm (102.4-196.9 inches). The diameter of proximal constant diameter section 26 may be about 0.01-0.04 inches, or about 0.015-0.030 inches, or about 0.023 inches.

Second tapered section 24 may have a length (represented in Figure 1 as dimension D2) of about 1-50cm (0.4-19.7 inches), or about 2-50cm (0.8-19.7 inches), or about 2-15cm (0.8-5.9 inches), or about 2-10cm (0.8-3.9 inches), or about 5 cm (2 inches). The diameter of second tapered section 24 may vary from a diameter that is equal to or approximately equal to the diameter of proximal constant diameter section 26 to a diameter that is equal to or approximately equal to the diameter of intermediate constant diameter section 22. The transition in diameter may be a linear taper or non-linear taper. For example, the tapering of second tapered section 24 may be a parabolic taper, a curvilinear taper, a straight taper, a function of a non-linear equation (e.g., a second order equation, a third order equation, a fourth order equation, or the like), or the like. The taper may be constant along the length of second tapered section 24 or the taper may vary along the length. In some embodiments, the taper may include one or more steps in diameter.

Intermediate constant diameter section 22 may have a length (represented in Figure 1 as dimension D3) of about 0.5-20cm (0.2-7.9 inches), or about 1-10cm (0.4-4 inches), or about 2.5-7.5cm (1-3 inches). The diameter of intermediate constant diameter section 22 may be about 0.001-0.04 inches, or about 0.005-0.015 inches, or about 0.01 inches.

First tapered section 20 may have a length (represented in Figure 1 as dimension D4) of about 0.1-10cm (0.04-4 inches), or about 0.5-10cm (0.2-4 inches), or about 0.5-3cm (0.2-1.2 inches), or about 1 cm (0.4 inches). The diameter of first tapered section 20 may vary from a diameter that is equal to or approximately equal to the diameter of intermediate constant diameter section 22 to a diameter that is equal to or approximately equal to the diameter of distal constant diameter section 18. The transition in diameter may be a linear taper or non-linear taper. For example, the tapering of first tapered section 20 may be a parabolic taper, a curvilinear taper, a straight taper, a function of a non-linear equation (e.g., a second order equation, a third order equation, a fourth order equation, or the like), or the like. The taper may be constant along the length of first tapered section 20 or the taper may vary along the length. In some embodiments, the taper may include one or more steps in diameter.

Distal constant diameter section 18 may have a length (represented in Figure 1 as dimension D5) of about 0.1-5cm (0.04-2 inches), or about 0.1-2.5cm (0.04-1 inches), or about 1.5cm (0.6 inches). The diameter of distal constant diameter section 18 may be about 0.001-0.02 inches, or about 0.001-0.008 inches, or about 0.005 inches. In some embodiments, the ratio of length of distal constant diameter section 18 to the diameter of distal constant diameter section 18 may be in the range of about 100:1-1500:1, or about 200:1-1200:1, or about 800:1-1200:1.

In some embodiments, distal constant diameter section 18 may have a cross-sectional shape that is substantially round. In other embodiments, distal constant diameter section 18 may be flattened or otherwise have a non-circular cross-sectional shape.

Forming core wire 12 may include a grinding technique such as through the use of a centerless grinder. In some embodiments, a standard centerless grinder may be used to form core wire 12. In other embodiments, the centerless grinding teachnique may be modified to use a thinner cutting wheel, which may allow for greater precision and/or for the creature of increasing and decreasing tapers along the length of core wire 12. In some of these and in other embodiments, different grinding modes may be used to form the desired configuration of core wire 12 such as "OD" mode.

Guidewires for using in coronary interventions are often designed to have a relatively stiff proximal section for providing "pushability" and a relatively flexible distal region and/or tip. In addition, coronary guidewires may be designed to avoid kinking or prolapsing when being advanced through a blood vessel. A kinked or prolapsed guidewire may not be fully functional and may not be suitable for guiding other diagnostic and/or therapeutic devices to a target region.

Guidewire 10 may find utility for use in endoscope interventions such as accessing the common bile duct (and/or the biliary tree), the pancreatic duct (and/or the pancreatic tree), or the like. Unlike coronary guidewires, guidewire 10 is designed to have one or more pre-determined inflection points where guidewire 10 may prolapse or otherwise form a loop. This design may desirably allow guidewire 10 to be used in endoscopic interventions or otherwise be used along the biliary or pancreatic tree. For example, the ability of guidewire 10 to form a predictable, pre-determined loop configuration may desirably impact the ability of guidewire 10 to cannulate anatomical structures such as the papilla of Vater, gain access to body lumens such as the common bile duct and/or the pancreatic duct, or otherwise perform endoscopic interventions.

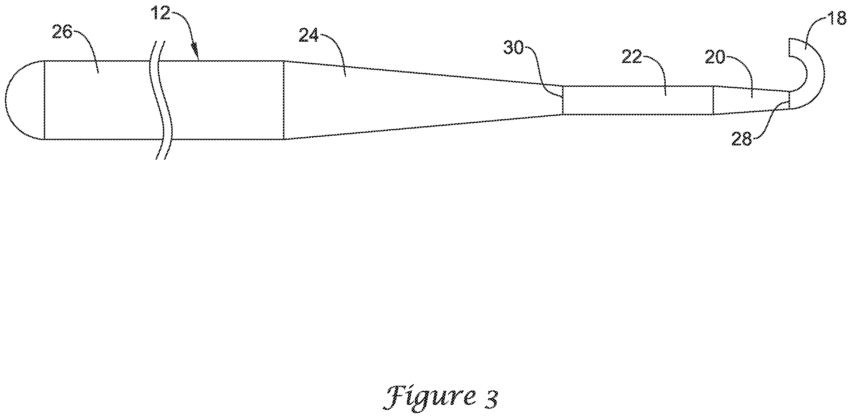

The design of core wire 12 may aid in guidewire 10 being able to form a predictable, pre-determined loop configuration. For example, a first inflection point 28 may be defined where distal constant diameter section 18 and first tapered section 20 meet. Because of the length of distal constant diameter section 18, first inflection point 28 may be positioned relatively close to the distal end of guidewire 10. When guidewire 10 is subjected to a suitable longitudinal force, distal constant diameter section 18 may prolapse or otherwise form a loop as shown in Figure 3. In at least some embodiments, the amount of force may be relatively low. For example, guidewire 10 may form the loop at first inflection point 28 when subjected to about 20-200g of longitudinal force, or about 20-200g of longitudinal force. In some embodiments, the amount of longitudinal force that may cause guidewire 10 to form a loop at first inflection point 28 may be about 1.1-2.0 times the insertion force (e.g., the force needed to insert guidewire 10 into a catheter, endoscope, or another device), or about 1.2-1.5 times the insertion force, or about 1.25 times the insertion force.

The ability to form a relatively tight loop with a relatively small amount of force may be desirable. For example, cannulation methods for accessing the bile duct may be challenging. By quickly forming a tight loop, a clinician may be able to more efficiently navigate anatomical structures such as the papilla of Vater. Furthermore, because first inflection point 28 is positioned close to the distal end of guidewire 10, the loop formed when guidewire 10 may be shorter, more tightly associated with the rest of the guidewire (e.g., the radius of curvature may be kept to a minimum), and less force may be applied to the surrounding anatomy. Accordingly, guidewire 10 may form a loop more easily, may have a better "feel" for clinicians, and may also be able to revert back to a linear state more easily and/or with less trauma to the surrounding tissue. In addition, the guidewire 10 may be able to "flick" or otherwise snap back to a more "unlooped" state so that guidewire 10 can continue to advance through the anatomy.

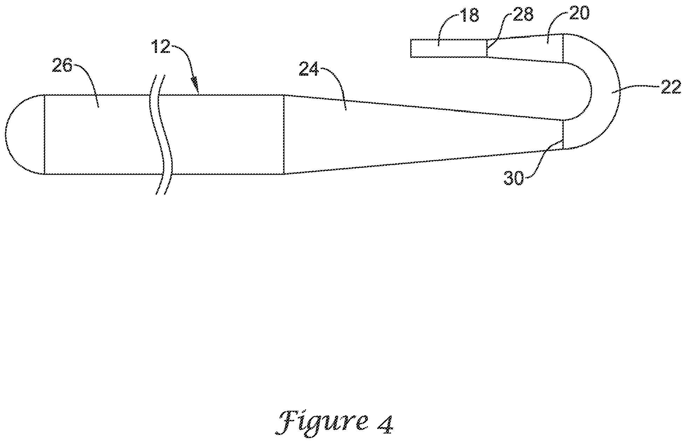

In at least some embodiments, guidewire 10 may include a plurality of inflection points. For example, a second inflection point 30 may be defined where intermediate constant diameter section 22 and second tapered section 24 meet. When guidewire 10 is subjected to a suitable longitudinal force, the sections of core wire 12 distal of second inflection point 30 may prolapse or otherwise form a loop as shown in Figure 4. In at least some embodiments, the amount of force may greater than that of first inflection point 28. For example, guidewire 10 may form the loop at second inflection point 30 when subjected to about 250-700g of longitudinal force. In some embodiments, the amount of longitudinal force that may cause guidewire 10 to form a loop at second inflection point 30 may be about 1.75-2.5 times the insertion force (e.g., the force needed to insert guidewire 10 into a catheter, endoscope, or another device), or about 1.8-2.2 times the insertion force, or about 2 times the insertion force.

By having inflection points 28/30, guidewire 10 may form a loop at a specific location along the length thereof, with a specific force, and with a controlled (and/or reduced) circumferential force on the body lumen. In at least some embodiments, inflection points 28/30 may be defined where a constant diameter section meets a tapered section. However, other arrangements are contemplated. For example, other structural modifications may also be utilized to define an inflection point such as stiffened regions of core wire 12, structures secured to core wire 12 to impart stiffness, changes in the location and/or composition of the sections of core wire 12, changes in the composition and/or location of sheath 14 and/or tip member 16, or the like.

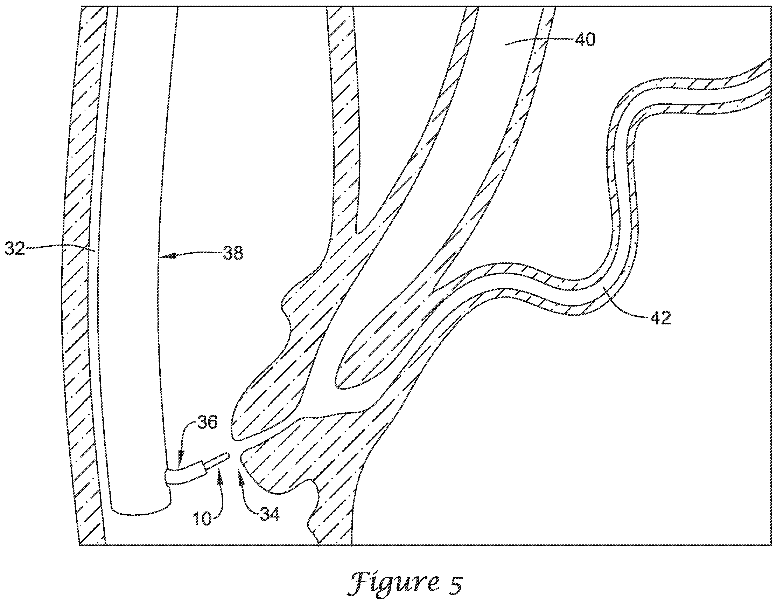

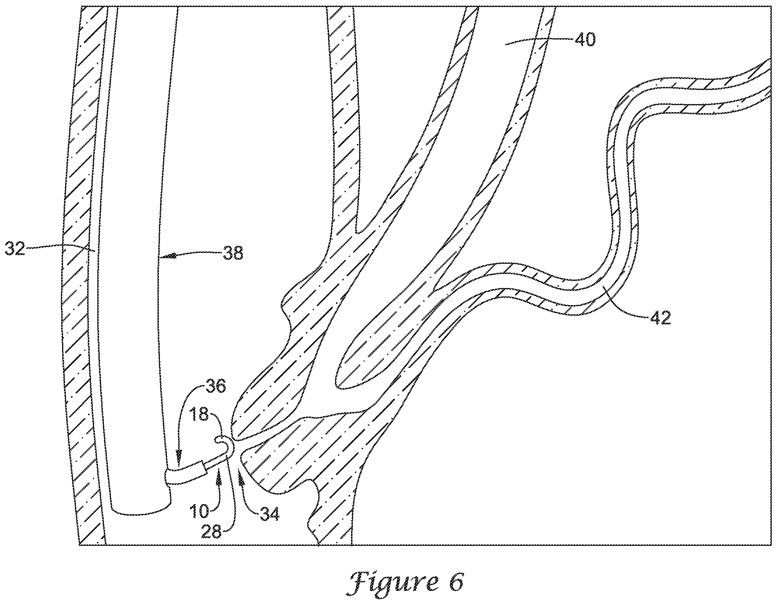

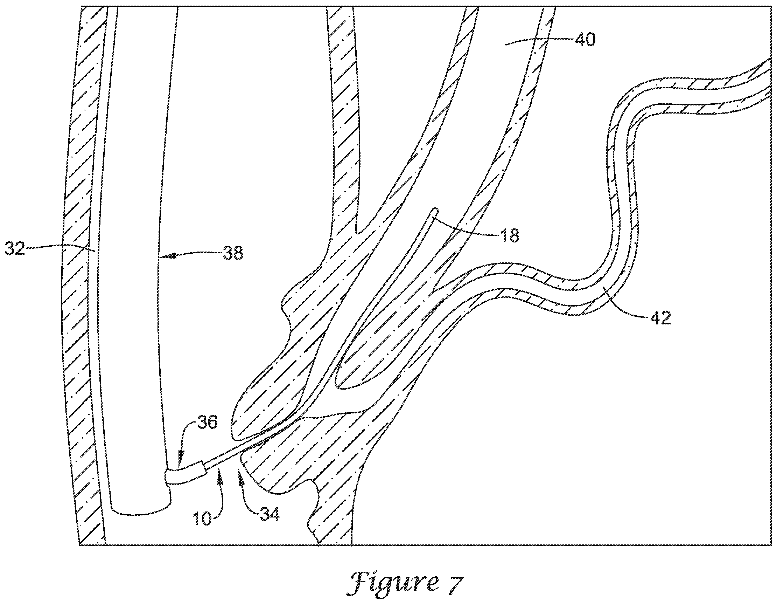

In use, guidewire 10 may be advanced through a body lumen such as the duodenum 32 to a position adjacent to the papilla of Vater 34 as shown in Figure 5. This may include the use of a cannulation or guide catheter 36 and/or an endoscope 38. Guidewire 10 may be advanced out from guide catheter 36 and into engagement with the papilla of Vater 34. If a suitable amount of resistance is encountered, guidewire 10 may prolapse or form a loop a first inflection point 28 as shown in Figure 6. Guidewire 10 may be advanced through the papilla of Vater 34 to a suitable diagnostic and/or treatment site such as along the biliary tract (e.g., the common bile duct 40) or the pancreatic tract (e.g., the pancreatic duct 42). For example, guidewire 10 is shown advanced into the common bile duct 40 in Figure 7.

The materials that can be used for the various components of guidewire 10 may include metals, metal alloys, polymers, metal-polymer composites, ceramics, combinations thereof, and the like, or other suitable materials. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b-*styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM™ (available from Neo-Metrics) and GUM METAL™ (available from Toyota). In some other embodiments, a superelastic alloy, for example a superelastic nitinol can be used to achieve desired properties.

In at least some embodiments, portions or all of core wire 12 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of guidewire 10 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of guidewire 10 to achieve the same result.

In some embodiments, a degree of Magnetic Resonance Imaging (MRI) compatibility is imparted into guidewire 10. For example, core wire 12, or portions thereof, may be made of a material that does not substantially distort the image and create substantial artifacts (i.e., gaps in the image). Certain ferromagnetic materials, for example, may not be suitable because they may create artifacts in an MRI image. Core wire 12, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

Referring now to core wire 12, the entire core wire 12 can be made of the same material along its length, or in some embodiments, can include portions or sections made of different materials. In some embodiments, the material used to construct core wire 12 is chosen to impart varying flexibility and stiffness characteristics to different portions of core wire 12. In embodiments where different portions of core wire 12 are made of different materials, the different portions can be connected using a suitable connecting technique and/or with a connector. For example, the different portions of core wire 12 can be connected using welding (including laser welding), soldering, brazing, adhesive, or the like, or combinations thereof. These techniques can be utilized regardless of whether or not a connector is utilized.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A medical guidewire (10), comprising:
a core wire (12) having a longitudinal axis, the core wire (12) including:
a distal constant diameter section (18) having a distal end and a proximal end, the distal end of the distal constant diameter section (18) defining a distal end of the core wire (12);
a first tapered section (20) having a distal end and a proximal end, the distal end of the first tapered section (20) being attached to the proximal end of the distal constant diameter section (18) such that a first inflection point (28) is defined where the distal end of the first tapered section (20) and the proximal end of the distal constant diameter section (18) meet;
an intermediate constant diameter section (22) having a proximal end and a distal end, the distal end of the intermediate constant diameter section (22) being attached to the proximal end of the first tapered section (20);
a second tapered section (24) having a proximal end and a distal end, the distal end of the second tapered section (24) being attached to the proximal end of the intermediate constant diameter section (22) such that a second inflection point (30) is defined where the distal end of the second tapered section (24) and the proximal end of the intermediate constant diameter section (22) meet; and
a proximal constant diameter section (26) having a proximal end and a distal end, the distal end of the proximal constant diameter section (26) being attached to the proximal end of the second tapered section (24);
wherein the core wire (12) is configured such that when a first predetermined longitudinal force is applied to the distal end of the distal constant diameter section (18) along the longitudinal axis, the distal constant diameter section (18) prolapses such that a first loop is defined about the first inflection point (28).

2. The guidewire of claim 1, wherein the core wire (12) is configured such that when the first loop is defined about the first inflection point (28), the first loop defines a distal extremity of the core wire (12), and when a second predetermined longitudinal force is applied to the distal extremity along the longitudinal axis, the intermediate constant diameter section (22) prolapses to define a second loop about the second inflection point (30).

3. The guidewire of claim 2, wherein the first predetermined longitudinal force is less than the second predetermined longitudinal force.

4. The guidewire of any one of claims 2-3, wherein the first predetermined longitudinal force is in the range of 20g to 200g.

5. The guidewire of any one of claims 2-4, wherein the second predetermined longitudinal force is in the range of 250g to 700g.

6. The guidewire of any one of claims 1-5, wherein the distal constant diameter section (18) has a length in the range of 0.1cm to 2.5 cm.

7. The guidewire of any one of claims 1-6, wherein the distal constant diameter section (18) has a diameter in the range of 0.025 mm to 0.203 mm (0.001 inches to 0.008 inches).

8. The guidewire of any one of claims 1-7, wherein the first tapered section (20) has a length in the range of 0.5cm to 3cm, wherein the intermediate constant diameter section (22) has a length in the range of 1cm to 10cm, and wherein the second tapered section (24) has a length in the range of 2cm to 50cm.

9. The guidewire of any one of claims 1-8, wherein the intermediate constant diameter section (22) has a diameter in the range of 0.127 mm to 0.381 mm (0.005 inches to 0.015 inches).

10. The guidewire of any one of claims 1-9, wherein the proximal constant diameter section (26) has a diameter in the range of 0.381 mm to 0.762 mm (0.015 inches to 0.030 inches).

11. The guidewire of any one of claims 1-10, wherein the ratio of the length of the distal constant diameter section (18) to the diameter of the distal constant diameter section (18) is in the range of 800:1 to 1200:1.

12. The guidewire of any one of claims 1-11, wherein the core wire (12) comprises a superelastic material.

13. The guidewire of claim 12, wherein the core wire (12) comprises a superelastic nickel-titanium alloy.

14. The guidewire of any one of claims 1-13, further comprising an outer sheath (14) disposed along at least a portion of the core wire (12).

15. The guidewire of any one of claims 1-14, further comprising a tip member (16) disposed along at least the distal constant diameter section (18).

## Patentansprüche

1. Medizinischer Führungsdraht (10), aufweisend:
einen Kerndraht (12) mit einer Längsachse, wobei der Kerndraht (12) umfasst:
einen distalen Abschnitt (18) mit konstantem Durchmesser, der ein distales Ende und ein proximales Ende aufweist, wobei das distale Ende des distalen Abschnitts (18) mit konstantem Durchmesser ein distales Ende des Kerndrahts (12) definiert;
einen ersten verjüngten Abschnitt (20) mit einem distalen Ende und einem proximalen Ende, wobei das distale Ende des ersten verjüngten Abschnitts (20) an dem proximalen Ende des distalen Abschnitts (18) mit konstantem Durchmesser derart angebracht ist, dass dort, wo das distale Ende des ersten verjüngten Abschnitts (20) und das proximale Ende des distalen Abschnitts (18) mit konstantem Durchmesser aufeinandertreffen, ein erster Biegepunkt (28) definiert wird;
einen Zwischenabschnitt (22) mit konstantem Durchmesser, der ein proximales Ende und ein distales Ende aufweist, wobei das distale Ende des Zwischenabschnitts (22) mit konstantem Durchmesser an dem proximalen Ende des ersten verjüngten Abschnitts (20) angebracht ist;
einen zweiten verjüngten Abschnitt (24) mit einem proximalen Ende und einem distalen Ende, wobei das distale Ende des zweiten verjüngten Abschnitts (24) an dem proximalen Ende des Zwischenabschnitts (22) mit konstantem Durchmesser derart angebracht ist, dass dort, wo das distale Ende des zweiten verjüngten Abschnitts (24) und das proximale Ende des Zwischenabschnitts (22) mit konstantem Durchmesser aufeinandertreffen, ein zweiter Biegepunkt (30) definiert wird; und
einen proximalen Abschnitt (26) mit konstantem Durchmesser, der ein proximales Ende und ein distales Ende aufweist, wobei das distale Ende des proximalen Abschnitts (26) mit konstantem Durchmesser an dem proximalen Ende des zweiten verjüngten Abschnitts (24) angebracht ist;
wobei der Kerndraht (12) derart ausgebildet ist, dass wenn eine erste vorgegebene Längskraft auf das distale Ende des distalen Abschnitts (18) mit konstantem Durchmesser entlang der Längsachse aufgebracht wird, der distale Abschnitt (18) mit konstantem Durchmesser derart prolabiert, dass um den ersten Biegepunkt (28) herum ein erster Bogen definiert wird.

2. Führungsdraht nach Anspruch 1, wobei der Kerndraht (12) derart eingerichtet ist, dass wenn der erste Bogen um den ersten Biegepunkt (28) herum definiert wird, der erste Bogen eine distale Extremität des Kerndrahts (12) definiert, und wenn eine zweite vorgegebene Längskraft auf die distale Extremität entlang der Längsachse aufgebracht wird, der Zwischenabschnitt (22) mit konstantem Durchmesser prolabiert, um einen zweiten Bogen um den zweiten Biegepunkt (30) herum zu definieren.

3. Führungsdraht nach Anspruch 2, wobei die erste vorgegebene Längskraft kleiner ist als die zweite vorgegebene Längskraft.

4. Führungsdraht nach einem der Ansprüche 2 bis 3, wobei die erste vorgegebene Längskraft im Bereich von 20 g bis 200 g liegt.

5. Führungsdraht nach einem der Ansprüche 2 bis 4, wobei die zweite vorgegebene Längskraft im Bereich von 250 g bis 700 g liegt.

6. Führungsdraht nach einem der Ansprüche 1 bis 5, wobei der distale Abschnitt (18) mit konstantem Durchmesser eine Länge im Bereich von 0,1 cm bis 2,5 cm hat.

7. Führungsdraht nach einem der Ansprüche 1 bis 6, wobei der distale Abschnitt (18) mit konstantem Durchmesser einen Durchmesser im Bereich von 0,025 mm bis 0,203 mm (0,001 Zoll bis 0,008 Zoll) hat.

8. Führungsdraht nach einem der Ansprüche 1 bis 7, wobei der erste verjüngte Abschnitt (20) eine Länge im Bereich von 0,5 cm bis 3 cm hat, wobei der Zwischenabschnitt (22) mit konstantem Durchmesser eine Länge im Bereich von 1 cm bis 10 cm hat, und wobei der zweite verjüngte Abschnitt (24) eine Länge im Bereich von 2 cm bis 50 cm hat.

9. Führungsdraht nach einem der Ansprüche 1 bis 8, wobei der Zwischenabschnitt (22) mit konstantem Durchmesser einen Durchmesser im Bereich von 0,127 mm bis 0,381 mm (0,005 Zoll bis 0,015 Zoll) hat.

10. Führungsdraht nach einem der Ansprüche 1 bis 9, wobei der proximale Abschnitt (26) mit konstantem Durchmesser einen Durchmesser im Bereich von 0,381 mm bis 0,762 mm (0,015 Zoll bis 0,030 Zoll) hat.

11. Führungsdraht nach einem der Ansprüche 1 bis 10, wobei das Verhältnis der Länge des distalen Abschnitts (18) mit konstantem Durchmesser zum Durchmesser des distalen Abschnitts (18) mit konstantem Durchmesser im Bereich von 800:1 bis 1200:1 liegt.

12. Führungsdraht nach einem der Ansprüche 1 bis 11, wobei der Kerndraht (12) ein superelastisches Material aufweist.

13. Führungsdraht nach Anspruch 12, wobei der Kerndraht (12) eine superelastische Nickel-Titan-Legierung aufweist.

14. Führungsdraht nach einem der Ansprüche 1 bis 13, ferner aufweisend einen Außenmantel (14), der zumindest entlang eines Teils des Kerndrahts (12) angeordnet ist.

15. Führungsdraht nach einem der Ansprüche 1 bis 14, ferner aufweisend ein Spitzenelement (16), das zumindest entlang des distalen Abschnitts (18) mit konstantem Durchmesser angeordnet ist.

## Revendications

1. Fil de guidage médical (10), comprenant :
un fil d'âme (12) ayant un axe longitudinal, le fil d'âme (12) incluant :
une section de diamètre constant distale (18) ayant une extrémité distale et une extrémité proximale, l'extrémité distale de la section de diamètre constant distale (18) définissant une extrémité distale du fil d'âme (12) ;
une première section conique (20) ayant une extrémité distale et une extrémité proximale, l'extrémité distale de la première section conique (20) étant fixée à l'extrémité proximale de la section de diamètre constant distale (18) de telle sorte qu'un premier point d'inflexion (28) est défini où l'extrémité distale de la première section conique (20) et l'extrémité proximale de la section de diamètre constant distale (18) se rencontrent ;
une section de diamètre constant intermédiaire (22) ayant une extrémité proximale et une extrémité distale, l'extrémité distale de la section de diamètre constant intermédiaire (22) étant fixée à l'extrémité proximale de la première section conique (20) ;
une seconde section conique (24) ayant une extrémité proximale et une extrémité distale, l'extrémité distale de la seconde section conique (24) étant fixée à l'extrémité proximale de la section de diamètre constant intermédiaire (22) de telle sorte qu'un second point d'inflexion (30) est défini où l'extrémité distale de la seconde section conique (24) et l'extrémité proximale de la section de diamètre constant intermédiaire (22) se rencontrent ; et
une section de diamètre constant proximale (26) ayant une extrémité proximale et une extrémité distale, l'extrémité distale de la section de diamètre constant proximale (26) étant fixée à l'extrémité proximale de la seconde section conique (24) ;
où le fil d'âme (12) est configuré de telle sorte que quand une première force longitudinale prédéterminée est appliquée à l'extrémité distale de la section de diamètre constant distale (18) le long de l'axe longitudinal, la section de diamètre constant distale (18) avance de telle sorte qu'une première boucle est définie au niveau du premier point d'inflexion (28).

2. Fil de guidage selon la revendication 1, où le fil d'âme (12) est configuré de telle sorte que quand la première boucle est définie au niveau du premier point d'inflexion (28), la première boucle définit une extrémité distale du fil d'âme (12), et quand une seconde force longitudinale prédéterminée est appliquée à l'extrémité distale le long de l'axe longitudinal, la section de diamètre constant intermédiaire (22) avance pour définir une seconde boucle au niveau du second point d'inflexion (30).

3. Fil de guidage selon la revendication 2, où la première force longitudinale prédéterminée est inférieure à la seconde force longitudinale prédéterminée.

4. Fil de guidage selon l'une quelconque des revendications 2-3, où la première force longitudinale prédéterminée est dans la plage de 20 g à 200 g.

5. Fil de guidage selon l'une quelconque des revendications 2-4, où la seconde force longitudinale prédéterminée est dans la plage de 250 g à 700 g.

6. Fil de guidage selon l'une quelconque des revendications 1-5, où la section de diamètre constant distale (18) a une longueur dans la plage de 0,1 cm à 2,5 cm.

7. Fil de guidage selon l'une quelconque des revendications 1-6, où la section de diamètre constant distale (18) a un diamètre dans la plage de 0,025 mm à 0,203 mm (0,001 pouce à 0,008 pouce).

8. Fil de guidage selon l'une quelconque des revendications 1-7, où la première section conique (20) a une longueur dans la plage de 0,5 cm à 3 cm, où la section de diamètre constant intermédiaire (22) a une longueur dans la plage de 1 cm à 10 cm et où la seconde section conique (24) a une longueur dans la plage de 2 cm à 50 cm.

9. Fil de guidage selon l'une quelconque des revendications 1-8, où la section de diamètre constant intermédiaire (22) a un diamètre dans la plage de 0,127 mm à 0,381 mm (0,005 pouce à 0,015 pouce).

10. Fil de guidage selon l'une quelconque des revendications 1-9, où la section de diamètre constant proximale (26) a un diamètre dans la plage de 0,381 mm à 0,762 mm (0,015 pouce à 0,030 pouce).

11. Fil de guidage selon l'une quelconque des revendications 1-10, où le rapport de la longueur de la section de diamètre constant distale (18) au diamètre de la section de diamètre constant distale (18) est dans la plage de 800 : 1 à 1200 : 1.

12. Fil de guidage selon l'une quelconque des revendications 1-11, où le fil d'âme (12) comprend un matériau superélastique.

13. Fil de guidage selon la revendication 12, où le fil d'âme (12) comprend un alliage nickel-titane superélastique.

14. Fil de guidage selon l'une quelconque des revendications 1-13, comprenant en outre une gaine externe (14) disposée le long d'au moins une partie du fil d'âme (12).

15. Fil de guidage selon l'une quelconque des revendications 1-14, comprenant en outre un élément de bout (16) disposé le long d'au moins la section de diamètre constant distale (18).
